# EUROPÄISCHE PATENTSCHRIFT

(11) **EP 1 694 288 B1**
(45) Veröffentlichungstag und Bekanntmachung des Hinweises auf die Patenterteilung: **09.04.2008**
(21) Anmeldenummer: 04802909.4
(22) Anmeldetag: 09.12.2004
(51) Int. Cl.: A61K 8/26, A61Q 1/04, A61Q 3/02

(54) **KOSMETISCHE ZUSAMMENSETZUNG ZUR ERZEUGUNG EINES KOSMETISCHEN ÜBERZUGS MIT EINEM METALLISCH UND MEHRFARBIG SCHILLERNDEN ERSCHEINUNGSBILD UND KÜNSTLICHER FINGERNAGEL**
COSMETIC PREPARATIONS FOR THE GENERATION OF A COSMETIC COVERING WITH A METALLIC AND MULTI-COLOURED SHIMMERING APPEARANCE AND ARTIFICIAL FINGERNAIL
COMPOSITION COSMETIQUE SERVANT A PRODUIRE UNE PELLICULE COSMETIQUE D'ASPECT MIROITANT METALLIQUE ET POLYCHROME ET ONGLE ARTIFICIEL

(30) Priorität: 10.12.2003 DE 10358091
(43) Veröffentlichungstag der Anmeldung: 30.08.2006
(73) Patentinhaber: Eckart GmbH, 90763 Fürth (DE)
(72) Erfinder: HENGLEIN, Frank, 90409 Nürnberg (DE); SCHUSTER, Thomas, 91207 Lauf (DE)
(74) Vertreter: Walcher, Armin
(86) Internationale Anmeldenummer: PCT/DE2004/002703
(87) Internationale Veröffentlichungsnummer: WO 2005/055965

(56) Entgegenhaltungen:
- EP-A- 0 826 745
- US-A- 4 321 087
- US-A1- 2003 175 225
- US-B1- 6 565 835
- J. SEUBERT, A. FETZ: "PVD Aluminum Pigments: Superior Brilliance for Coatings & Graphic Arts" 6. Januar 2000 (2000-01-06), XP002322107 Gefunden im Internet: URL:http://www.pcimag.com/CDA/ArticleInfor mation/coverstory/BNPCoverStoryItem/0,1848 ,3507,00.html>

## Beschreibung

Die Erfindung betrifft eine kosmetische Zusammensetzung zur Erzeugung eines kosmetischen Überzugs mit einem metallisch und mehrfarbig schillernden Erscheinungsbild.

Aus der WO 02/03913 ist ein Nagellack, der einem Fingernagel ein metallisches Erscheinungsbild verleiht, bekannt. Bei dieser bekannten Nagellack-Formulierung werden PVD-Pigmente verwendet. Weitere Farbeffekte, insbesondere ein mehrfarbiges Schillern, können mit diesem Nagellack nicht erhalten werden.

Von der Firma Flex (Santa Rosa, Kalifornien, USA) werden unter dem Handelsnamen Spectraflair® Pigmente angeboten. Der Aufbau und die Herstellung dieser Pigmente sind in Barbara Parker, "Advances in Interference Color", im Tagungsband Color Cosmetic Summit, Montreal (2003) beschrieben. Bei diesen Pigmenten handelt sich um mit PVD-Verfahren erzeugte, mehrschichtige Effektpigmente mit einem zentralen Kern aus Aluminium und darauf aufgebrachten Schichten aus MgF2. Auf diesen niedrig brechenden Schichten ist ein Gitter mit definiertem Abstand der Gitterstrukturen aufgeprägt. Bei einfallendem sichtbaren Licht kommt es bei jedem Effektpigment zu einem Prisma-artigen Beugungseffekt. Dieser führt in der Applikation - eine gleichmäßige planparallele Orientierung der Effektpigmente innerhalb eines beschichteten Überzuges auf einem Substrat vorausgesetzt - zu einem Regenbogenfarbenartigem Effekt der Beschichtung.

Weiterhin können Regenbogeneffekte auch durch Verwendung sogenannter "holografischer" Effektpigmente erzeugt werden. Unter diesem Begriff wird eine Reihe von Effektpigmente mit unterschiedlichem Aufbau verstanden. Beispielsweise versteht man hierunter sogenannte "Geometric Pigments", die von der Fa. Eckart, Fürth, Deutschland, vertrieben werden. Bei diesen "Geometric Pigments" sind mehrere Polymerschichten auf einen Aluminiumkern unter Verwendung von PVD-Verfahren aufgebracht worden. Diese Effektpigmente besitzen Schichtdicken bis zu 10 mm und sind insbesondere auf Grund dieser großen Schichtdicken für kosmetische Formulierungen nicht geeignet.

Die vorgenannten - zum Teil bereits im Handel erhältlichen - Effektpigmente mit Gitter- oder Hologrammstruktur besitzen alle den wesentlichen Nachteil, das es sich hierbei um relativ dicke Effektpigmente handelt. Dieser Nachteil begrenzt äußerst stark die Verwendung dieser Pigmente in kosmetischen Formulierungen.

Ein weiterer Nachteil ist die Tatsache, dass die genannten Effektpigmente über eine relativ große Teilchengröße, d.h. über einen großen Durchmesser verfügen, der im Bereich von über 25 µm liegt.

So könnten beispielsweise Spectraflair®-Pigmente in Nagellacken verwendet werden, um Nagellack-Überzüge mit Regenbogeneffekt und metallischem Erscheinungsbild zu erzeugen. Aufgrund der großen Dicke der Spectraflair®-Pigmente von etwa 1 mm (Barbara Parker, "Advances in Interference Color" im Tagungsband Color Cosmetic Summit, Montreal (2003)) und der großen Teilchengröße von etwa 25 µm bis 35 µm erzeugen diese Pigmente in einem Überzug beim Betrachter nicht den Eindruck eines durchgehenden metallischen Films, sondern werden von dem Betrachter vielmehr als einzelne Pigmente in Form eines "Glittereffektes" oder als "Sparkle-Effekt" wahrgenommen.

Ein derartiger Glitter- oder Sparkle-Effekt ist in kosmetischen Formulierungen wie Nagellacken jedoch nur bedingt erwünscht. In diesem Markt wird von den meisten Frauen eine Nagellackierung mit einer gewissen Eleganz bevorzugt, bei der die individuellen Pigmentteilchen bei der visuellen Wahrnehmung zu einem gleichmäßigen Metallfilm verschmolzen sind.

Der Regenbogenfarbeneffekt sollte auch nicht zu stark ausgeprägt sein, da die Nagellackierung andernfalls zu kitschig wirkt.

Es besteht mithin ein Bedarf an kosmetischen Formulierungen, die nach Aufbringung einen Überzug mit dezentem Regenbogenfarbeneffekt und elegantem Aussehen bereitstellen.

Ein weiter Nachteil der oben erwähnten Effektpigmente ist deren geringe Deckkraft. Die geringe Deckkraft ergibt sich durch zwei Effekte:
Zum einen handelt es sich bei den oben beschriebenen Effektpigmenten immer um solche, die nur zu einem relativ geringen Teil aus Aluminium bestehen. Bei diesen Effektpigmenten trägt jedoch nur der Aluminiumkern zur Deckkraft bei, da nur an dem Aluminiumkern das sichtbare Licht (400 bis 800 nm Wellenlänge) reflektiert wird. Je geringer der Anteil des Aluminiumkernes bei dem Effektpigment ist, desto geringer ist die Deckkraft. Die mangelhafte Deckkraft dieser Pigmente führt ferner zu einer geringen Ergiebigkeit in der kosmetischen Beschichtungszusammensetzung.
Zum anderen verhindern die hohen Schichtdicken dieser Effektpigmente eine günstige Stapelung der Pigmente im Anwendungsmedium. So sind beispielsweise die "Geometric Pigments" mit Schichtdicken von 10 mm im Nagellackbereich völlig ungeeignet, da sich diese Pigmente nicht mehr ausreichend orientieren können. Eine gute Orientierung, insbesondere eine im wesentlichen planparallele Ausrichtung zum Untergrund, beispielsweise einem Fingernagel, ist für die Erzielung eines guten visuellen Erscheinungsbildes erforderlich.

Des weiteren ist die Verwendung dieser Effektpigmente auf Grund der sehr teuren und aufwendigen Herstellungsverfahren in kosmetischen Zusammensetzungen zur Erzeugung von Überzügen mit metallischem Aussehen und mehrfarbigem Schillern (Regenbogeneffekt) unwirtschaftlich.

Regenbogeneffekte mit sehr hohem metallischen Aussehen und Glanz sowie guter Deckkraft können weiterhin durch Verwendung von Prägefolien erzielt werden. Hierbei werden metallisierte Kunststofffolien mit Gitterstruktur verwendet. Derartige Folien werden beispielsweise von der Fa. Kurz GmbH & Co. KG, Fürth, Deutschland, hergestellt und angeboten. Derartige Folien können jedoch nicht in kosmetischen Formulierungen verwendet werden.

Aufgabe der vorliegenden Erfindung ist es, eine kosmetische Zusammensetzung bereitzustellen, die nach der Applikation ein mehrfarbiges Schillern sowie einen glänzenden Metalleffekt mit dem Eindruck einer vorzugsweise geschlossenen Metallschicht erzeugt.

Die Ergiebigkeit der kosmetischen Zusammensetzung im Hinblick auf den gewünschten metallischen Regenbogeneffekt sollte möglichst hoch sein; d.h. die Pigmentierungshöhe bzw. der Gehalt an Pigment in der kosmetischen Formulierung sollte möglichst niedrig sein.

Die der Erfindung zugrunde liegende Aufgabe wird durch Bereitstellung einer kosmetischen Zusammensetzung zur Erzeugung eines kosmetischen Überzugs mit einem metallisch und mehrfarbig schillernden Erscheinungsbild, umfassend eine Flüssigphase und PVD-Aluminiumpigment, wobei das PVD-Aluminiumpigment Beugungsstrukturen mit etwa 5.000 bis etwa 20.000 Strukturelementen pro cm, einen metallischen Aluminiumanteil von 90 bis 100 Gew.-%, bezogen auf das Gewicht des Aluminiumpigments, besitzt und in der kosmetischen Zusammensetzung in einer Pigmentierungshöhe von 0,05 bis 5,0 Gew.-%, bezogen auf das Gesamtgewicht der kosmetischen Zusammensetzung, enthalten ist, gelöst.

Bevorzugte Weiterbildungen sind in den Unteransprüchen angegeben.

Die Aufgabe wird ferner durch Bereitstellung eines künstlichen Fingernagels gemäß Anspruch 27 gelöst. Im Sinne der Erfindung wird unter dem Begriff "künstlicher Fingernagel" auch ein künstlicher Fußnagel verstanden.

Unter PVD-Aluminiumpigment wird im Sinne der Erfindung verstanden, dass das Aluminiumpigment unter Verwendung eines PVD-Verfahrens (PVD: physical vapour deposition) hergestellt worden ist. Mittels PVD-Verfahren lassen sich sehr dünne Aluminiumpigmente mit einer äußerst glatten und spiegelnden Oberfläche herstellen. PVD-Verfahren zur Herstellung von Aluminiumpigmenten sind dem Fachmann beispielsweise aus der EP 0 826 745 bekannt, die hiermit unter Bezugnahme aufgenommen ist.

Die Herstellung von Aluminiumpigmenten aus Aluminium mit Beugungsstrukturen ist in der US 5,624,076 beschrieben. Diese Pigmente, die auch als geprägte Pigmente bezeichnet werden, weisen den großen Vorteil auf, dass sie nur aus sehr dünnem Aluminium mit einer Primärschichtdicke von ca. 25 bis 60 nm bestehen.

Die Beugungsstrukturen weisen gemäß der Lehre der US 5,624,076 5.000 bis 11.000 Gitterrillen pro cm auf.

Das Pigment wird gemäß der Lehre der US 5,624,076 hergestellt, indem einer Polymerfolie eine Gitterstruktur aufgeprägt und diese anschließend im Hochvakuum mit Aluminium bedampft wird. Danach wird - wie bei der Herstellung metallischer Effektpigmente durch PVD-Verfahren üblich - das Aluminium von der Polymerfolie entfernt und die Folie zu Pigmenten zerkleinert. Ein Verfahren zur Herstellung geprägter Aluminiumpigmente ist in der US 5,624,076 beschrieben, die hiermit unter Bezugnahme aufgenommen wird.

Mit diesem Verfahren können auch Beugungsstrukturen erzeugt werden, die bis zu 20.000 Beugungselemente pro cm aufweisen. Im Sinne der Erfindung wird für die PVD-Aluminiumpigmente mit Beugungsstrukturen im folgenden auch der Begriff "geprägte Aluminiumpigmente" verwendet.

Die Beugungsstrukturen sind vorzugsweise im Wesentlichen parallel zueinander angeordnete Rillen, d.h. durch Erhebungen oder Berge voneinander beabstandete Täler. Es ist natürlich auch möglich, andere Beugungsstrukturen auf den Aluminiumpigmenten zu verwenden. Beispielsweise können die Beugungsstrukturen in Form von ineinander angeordneten konzentrischen Rillenstrukturen oder spiralartig angeordnete Rillenstrukturen sein. Wesentlich ist lediglich, dass die Beugungsstrukturen beim Betrachter den gewünschten optischen Effekt in Form eines mehrfarbigen Schillerns oder eines Regenbogenfarbeneffektes hervorrufen.

Vorzugsweise sind die Beugungsstrukturen als Reflexionsgitter ausgebildet.

Es hat sich nunmehr völlig überraschend gezeigt, dass sich diese geprägten Aluminiumpigmente hervorragend zur Verwendung in kosmetischen Formulierungen eignen.

Die geprägten Aluminiumpigmente werden bevorzugt in stark verdünnten Dispersionen mit organischen Lösemitteln gehandhabt, um starke Agglomeration zu vermeiden.

Die Erfindung betrifft mithin eine kosmetische Zusammensetzung, welche nach Aufbringung einen Überzug mit vorzugsweise geschlossen erscheinendem Metallfilm, der ein farbiges Schillern aufweist, bereitstellt. Das Erscheinungsbild kann auch als metallischer Regenbogenfarbeneffekt beschrieben werden. Unter dem Begriff "Regenbogenfarbeneffekt" ist im Sinne der Erfindung nicht zu verstehen, dass eine völlige spektrale Zerlegung des sichtbaren Lichtes erfolgen muss. Vielmehr ist gemeint, dass der Betrachter bei der aufgebrachten erfindungsgemäßen Beschichtung ein mehrfarbiges Schillern wahrnimmt.

Unter einer kosmetischen Zusammensetzung werden im Sinne der Erfindung sämtliche kosmetischen Formulierungen verstanden, die eine Flüssigphase aufweisen, die erfindungsgemäß zu verwendende geprägte Aluminiumpigmente enthalten und auf im Wesentlichen flächigen Untergründen aufgetragen werden. Vorzugsweise werden die kosmetischen Zubereitungen aus der Gruppe ausgewählt, die aus flüssigem Make-up, flüssigem Eyeliner, flüssigem Lidschatten, Körperlotion, Parfüm, Lipgloss und Nagellack besteht.

Die kosmetische Zusammensetzung kann gemäß einer bevorzugten Ausführungsform Additive und/oder Zusatzstoffe wie beispielsweise Antioxidantien, Farbmittel, UV-Absorber, Emulgatoren, Vitamine, Duftstoffe, Medikamente, Thixotropiemittel und/oder Füllstoffe enthalten.

Vorzugsweise ist die kosmetische Zusammensetzung ein Nagellack oder eine Lipgloss-Formulierung.

Bei dem bei der erfindungsgemäßen kosmetischen Zusammensetzung verwendeten geprägten Aluminiumpigment tritt das metallische und mehrfarbige Erscheinungsbild oder der Regenbogenfarbeneffekt nach Auftragen der kosmetischen Zusammensetzung auf einen flächigen Untergrund auf. In der kosmetischen Zusammensetzung selbst ist der Effekt nicht notwendigerweise sichtbar, da hier die geprägten Aluminiumpigmente noch statistisch in der kosmetischen Zusammensetzung verteilt sind. Nach dem Auftragen der erfindungsgemäßen kosmetischen Zusammensetzung kommt es zu einer vorzugsweise planparallelen Orientierung zum Untergrund, beispielsweise auf natürlichen oder künstlichen Fingernägeln, auf Lippen, Augenlidern, Wangen oder anderen flächigen Körperbereichen. Um diese Orientierung der geprägten Aluminiumpigmente zu ermöglichen, ist es erforderlich, dass die kosmetische Zusammensetzung eine Flüssigphase aufweist.

Kosmetische Zusammensetzungen, die keine Flüssigphase enthalten, eignen sich vorwiegend nicht, da eine Flüssigphase erforderlich ist, um eine Orientierung der geprägten Aluminiumpigmente und damit den gewünschten metallischen und mehrfarbig schillernden Effekt in dem aufgebrachten Überzug zu ermöglichen.

Das heißt, für eine Orientierung der geprägten Aluminiumpigmente ist ein ausreichender Feuchtigkeitsgehalt in der kosmetischen Zusammensetzung nötig, um nach dem Auftragen der Zusammensetzung und Verdampfen des Lösemittels eine Orientierung der Effektpigmente zu ermöglichen. Ferner kann die Orientierung der geprägten Aluminiumpigmente verbessert bzw. unterstützt werden, wenn während des Auftragens der kosmetischen Zusammensetzung eine mechanische Einwirkung erfolgt. Dies ist beispielsweise beim Auftragen eines Nagellack mit einem Pinsel auf natürliche oder künstliche Fingernägel gegeben. Beim Aufbringen eines Lipgloss auf die Lippen wird die Orientierung beispielsweise durch die Auftrags- bzw. Rollkugel des Lipgloss-Auftragsstiftes unterstützt. Alternativ kann der Lipgloss auch mittels Pinselauftrag auf die Lippen aufgebracht werden.

Als Flüssigphase werden vorzugsweise flüssige Komponenten verwendet, die für die jeweilige Applikation gesundheitlich unbedenklich sind. Das heißt, bei einem Nagellack können organische Lösemittel verwendet werden, die bei einem Lipgloss gegebenenfalls nur eingeschränkt oder gar nicht verwendet werden können. Der Fachmann kann jedoch ohne weiteres eine für den jeweiligen Verwendungszweck geeignete Flüssigphase, beispielsweise ein organisches Lösemittel, Wasser, Öl, Gel oder geeignete Mischungen davon auswählen.

Eine erfindungsgemäße Nagellackzusammensetzung besteht in der Regel aus für diesen Verwendungszweck nicht-toxischen Komponenten und ist geeignet, einen Filmüberzug mit metallisch wirkendem Regenbogeneffekt auf natürlichen oder künstlichen Fingernägeln auszubilden. Der Begriff "nicht-toxisch" bezieht sich hierbei im allgemeinen auf die in der EG Richtlinie 76/768 aufgeführten Stoffe.

Eine Nagellackzusammensetzung enthält vorzugsweise ein Lösemittel, wenigstens eine filmbildende Komponente sowie geprägtes Aluminiumpigment, wobei das Aluminiumpigment Beugungsstrukturen mit etwa 5.000 bis etwa 20.000 Strukturelementen pro cm, eine Schichtdicke von etwa 20 bis etwa 140 nm, vorzugsweise von etwa 20 nm bis etwa 100 nm, weiter bevorzugt von etwa 25 nm bis etwa 80 nm, aufweist und einen metallischen Aluminiumanteil von 90 bis 100 Gew.-%, bezogen auf das Gewicht des Aluminiumpigments, besitzt.

Die filmbildende Komponente umfasst bevorzugt Nitrocellulose und deren geeigneten, nicht-toxischen Derivate sowie Celluloseacetatbutyrat. Vorzugsweise hat Nitrocellulose ein Molekulargewicht von wenigstens 56.000 g/mol, weiter bevorzugt von wenigstens 112.000 g/mol.

Das geprägte Aluminiumpigment wird vorzugsweise in Form einer Dispersion in einem organischen Lösemittel bei der Herstellung einer kosmetischen Zusammensetzung verwendet. Dabei kann der Feststoffgehalt der einer kosmetischen Zusammensetzung zuzusetzenden Dispersion 1 bis 15 Gew.-%, bevorzugt 3 bis 10 Gew.-%, betragen. Bei höheren Konzentrationen an geprägten Aluminiumpigmenten besteht die Gefahr einer Agglomeration der Aluminiumpigmente.

Bei der Wahl der Lösemittel ist man bei einer Nagellackzusammensetzung sehr frei, solange die Lösemittel für diesen Anwendungsbereich geeignet und zugelassen sind. Beispielsweise können Toluol, n-Propylacetat, Ethylacetat, Isopropylalkohol, Glycolether oder n-Butylacetat verwendet werden. Bevorzugt für Nagellackzusammensetzungen ist Ethylacetat.

Als organische Lösemittel in der Nagellackzusammensetzung können aber auch weitere Lösemittel wie beispielsweise Hexan, Heptan, Cyclopentan, Cyclohexan, zyklische Ether wie z.B. Tetrahydrofuran oder 1,4-Dioxan, Ethanol, iso-Propylacetat, , Cellosolveacetat, Ethylcellosolve, Butylcellosolve und Gemischen hiervon.

Der Lösemittelgehalt der erfindungsgemäßen Nagellackzusammensetzungen beträgt vorzugsweise 50 bis 90 Gew.-%, weiter bevorzugt 60 bis 85 Gew.-% und noch weiter bevorzugt 65 bis 75 Gew.-%, jeweils bezogen auf das Gesamtgewicht der Nagellackzusammensetzung.

Gemäß einer bevorzugten Weiterbildung der Erfindung kann die Nagellackzusammensetzung einen Weichmacher und/oder ein Dispergierungsmittel enthalten.

Weichmacher machen insbesondere das Bindemittel weicher und besser verarbeitbar. Es kann ein Weichmacher oder aber auch eine Kombination mehrerer Weichmacher verwendet werden. Beispiele für derartige Weichmacher sind:
Campher, Rizinusöl, Ester der Zitronen-, Stearin-, Öl-, Phthal-, und Benzoesäure.

Als Phthalate werden vorzugsweise Dibutylphthalat, Diethylphthalat, Diamylphthalat, Dioctylphthalat und Dibutoxyethylphthalat oder Mischungen davon verwendet. Es können aber auch andere Weichmacher wie beispielsweise Glyceryltriacetat, Glyceryltripropionat, Glyceryltribenzoat, Dibutyltartrat, Benzylbenzoat, Triphenylphosphat, Butylacetylriconeat, Butylstearat, Triethylcitrat, Acetyltributylcitrat, Saccharoseacetatisobutyrat oder Gemische davon verwendet werden.

Gemäß einer weiteren Ausführungsform der Erfindung können Dispergieradditive zugesetzt werden, um die geprägten Aluminiumpigmente und gegebenenfalls weiter verwendete Effekt- und/oder Farbpigmente besser zu dispergieren. Bevorzugte Beispiele derartiger Dispergiermittel sind Montmorillonit-Tone wie z.B. Bentonite und insbesondere Stearylalkoniumhectorit oder Stearylalkoniumbentonit. Als Dispergiermittel kann auch polymerer Harnstoff, optional in Kombination mit Bentoniten, verwendet werden.

Weitere Zusätze umfassen andere Effektpigmente und/oder Farbmittel wie Farbpigmente und/oder Farbstoffe. Effektpigmente können insbesondere Perlglanzpigmente sein. Diese werden vorzugsweise jedoch in relativ geringen Konzentrationen bis zu etwa 1 Gew.-% zugegeben, um den metallischen Regenbogenfarbeneffekt nicht zu beeinträchtigen.

Auch Farbpigmente können gemäß einer bevorzugten Weiterbildung der Erfindung zugegeben werden. Vorzugsweise werden die Farbpigmente ebenfalls in kleinen Konzentrationen bis zu etwa 3 Gew.-%, bevorzugt bis zu etwa 1 Gew.-% zugegeben. Die Farbpigmente umfassen beispielsweise TiO2, Eisenoxide (rot, schwarz, gelb), Chromoxid für Grüntöne, Ultramarin Blau oder in der Kosmetik typische Verlackungspigmente.

Weiterhin kann die erfindungsgemäße Nagellackzusammensetzung weitere Additive wie Thixotropiemittel, UV-Absorber, Antioxidantien, Farbmittel, Emulgatoren, Vitamine, Duftstoffe, Füllstoffe oder Medikamente enthalten. Diese Additive sind im Stand der Technik bekannt und werden gegebenenfalls in den üblichen Mengen dazugegeben.

Bevorzugt enthält eine erfindungsgemäße Nagellackzusammensetzung, die vorzugsweise aus nicht-toxischen Komponenten besteht, geprägtes Aluminiumpigment, bestehend aus gitterartig geprägtem Aluminium mit 5.000 bis 20.000 Gitterstrukturen pro cm, vorzugsweise 7.000 bis 15.000 Beugungsstrukturen pro cm, mit einer Primärschichtdicke von 20 bis 140 nm, vorzugsweise von etwa 20 nm bis etwa 100 nm, weiter bevorzugt von etwa 30 nm bis etwa 80 nm, und einem metallischen Aluminiumanteil von 90 bis 100 Gew.-%, bezogen auf das Gesamtgewicht des Aluminiumpigments, in einer Pigmentierungshöhe von 0,05 bis maximal 5 Gew.-%, bezogen auf das Gesamtgewicht des Nagellacks. Weiter bevorzugt ist eine Pigmentierungshöhe von 0,1 bis 2 Gew.-%, bezogen auf das Gesamtgewicht des Nagellacks.

Besonders bevorzugt enthält die Nagellackzusammensetzung nicht-toxische Komponenten, die Nitrocellulose mit einem Molekulargewicht von wenigstens 56.000 g/mol, Lösemittel, Weichmacher, Dispergierungsmittel und geprägtes Aluminiumpigment mit 5.000 bis 20.000 Beugungsstrukturen pro cm, vorzugsweise 7.000 bis 15.000 Beugungsstrukturen pro cm, mit einer Primärschichtdicke von 20 bis 140 nm, vorzugsweise von etwa 20 nm bis etwa 100 nm, weiter bevorzugt von etwa 30 nm bis etwa 80 nm, und einem metallischen Aluminiumanteil von 90 bis 100 Gew.-%, bezogen auf das Gesamtgewicht des Aluminiumpigments, in einer Pigmentierungshöhe von 0,05 bis 5 Gew.-%, bezogen auf das Gesamtgewicht des Nagellacks. Weiter bevorzugt ist eine Pigmentierungshöhe von 0,1 bis 2 Gew.-%, bezogen auf das Gesamtgewicht des Nagellacks.

Überraschenderweise hat sich gezeigt, dass das geprägte Aluminiumpigment in der kosmetischen Zusammensetzung mit sehr hoher Ergiebigkeit, d.h. hervorragender Deckkraft verwendet werden kann. Beschichtungszusammensetzungen mit diesem geprägten Aluminiumpigment weisen eine äußerst hohe Deckkraft auf. Daher ist das geprägte Aluminiumpigment vorteilhafterweise in sehr niedrigen Pigmentierungshöhen in der erfindungsgemäßen kosmetischen Zusammensetzung verwendbar. Der Gehaltan geprägtem Aluminiumpigment in der kosmetischen Zusammensetzung beträgt 0,05 bis 5,0 Gew.-%, bezogen auf das Gesamtgewicht der kosmetischen Zusammensetzung, vorzugsweise 0,1 bis 2,0 Gew.-%.

Die geprägten Aluminiumpigmente besitzen eine Primärschichtdicke von vorzugsweise etwa 20 bis 140 nm und unterscheiden sich mithin deutlich von den Dicken konventionell durch Naßmahlung hergestellter Aluminiumpigmente, die Dicken im Bereich von etwa 200 bis 1.000 nm aufweisen. Die geprägten Aluminiumpigmente sind auf Grund ihrer geringen Dicke so flexibel, dass sie sich perfekt an einen Untergrund anpassen, d.h. sich quasi an den Untergrund anschmiegen.

Unter der Primärschichtdicke wird hier die tatsächliche Schichtdicke des Aluminiums verstanden. Sie beträgt bei den in den erfindungsgemäßen Beschichtungszusammensetzungen verwendeten geprägten Aluminiumpigmenten vorzugsweise 20 bis 140 nm, weiter bevorzugt 20 bis 100 nm, noch weiter bevorzugt 20 bis 80 nm. Weiterhin bevorzugt sind Primärschichtdicken von 25 bis 75 nm oder 30 bis 65 nm.

Die Sekundärschichtdicke gibt hingegen den Abstand der Berggipfel auf den einander gegenüberliegenden Seiten eines geprägten Aluminiumpigmentes wieder.

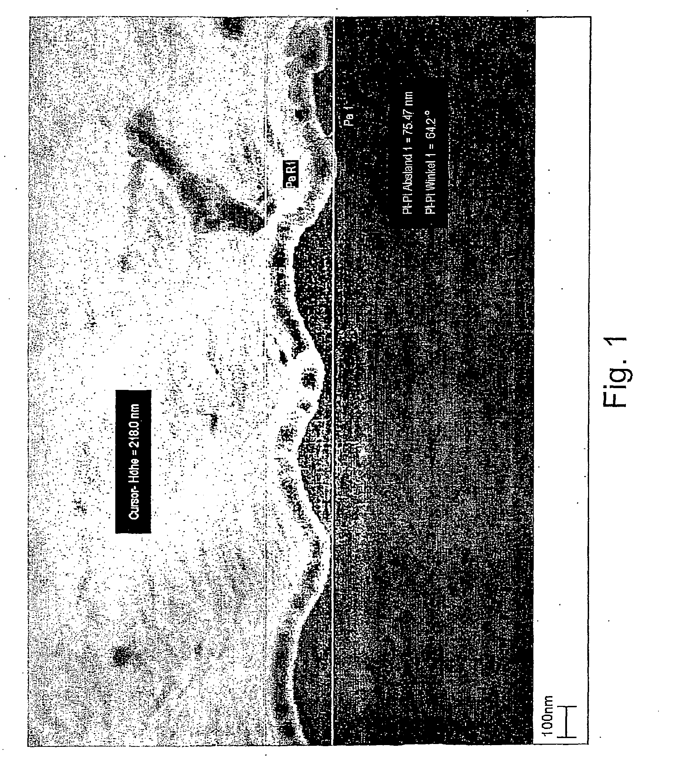

Fig. 1 zeigt eine REM-Aufnahme eines geprägten PVD-Aluminiumpigmentes. In Fig. 1 ist die Beugungsstruktur eines geprägten PVD-Aluminiumpigmentes in 50.000-facher Vergrößerung zu sehen. Fig. 1 veranschaulicht ferner, was vorliegend unter Primärschichtdicke und Sekundärschichtdicke zu verstehen ist. Die Primärschichtdicke ist die tatsächliche Dicke des Aluminiumpigmentes zwischen zwei einander gegenüberliegenden Punkten auf der Ober- und Unterseite des Aluminiumpigmentes. In Fig. 1 sind dies die Punkte PaR1 auf der Oberseite und Pa1 auf der Unterseite des Aluminiumpigmentes. Die Sekundärschichtdicke ist die Dicke des Pigmentes, die dem Abstand der weißen Linien entspricht, die jeweils entlang der Berggipfel auf der Ober- und Unterseite des Pigmentes angeordnet sind.

Die Sekundärschichtdicke der Effektpigmente der erfindungsgemäßen Beschichtungszusammensetzung beträgt vorzugsweise 150 bis 400 nm, bevorzugt 175 bis 350 nm, weiter bevorzugt 200 bis 300 nm und noch weiter bevorzugt 220 bis 280 nm.

Die geprägten Aluminiumpigmente können grundsätzlich zwei Extrempositionen zueinander einnehmen, nämlich eine "Berg-zu-Tal"-Anordnung oder eine "Bergzu-Berg"-Anordnung.

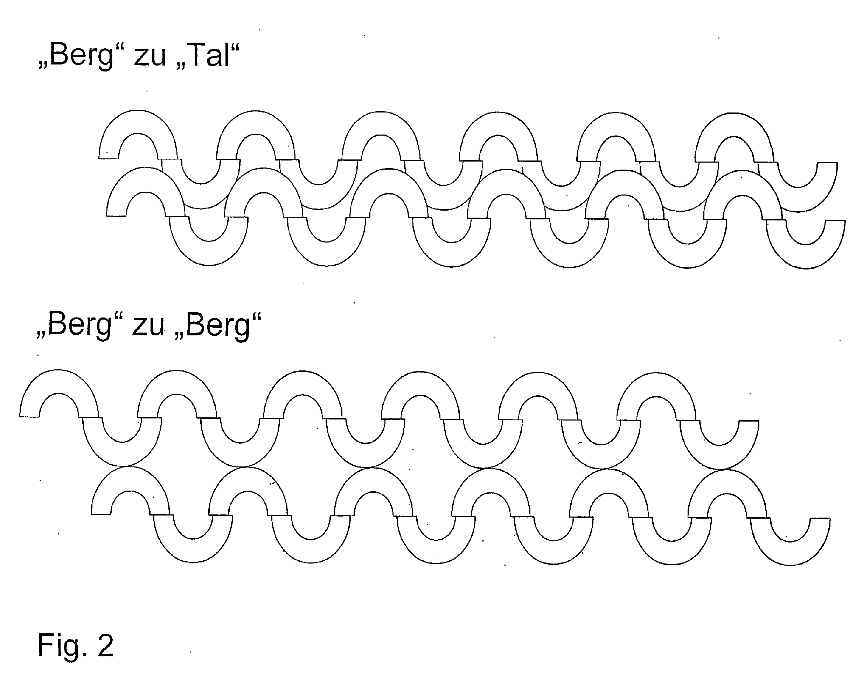

In Fig. 2 ist schematisch veranschaulicht, was vorliegend unter einer "Berg-zu-Tal"-Anordnung bzw. einer "Berg-zu-Berg"-Anordnung zu verstehen ist.

Bei einer "Berg-zu-Tal"-Anordnung wird der Abstand der geprägten Aluminiumpigmente zueinander sehr gering. Die Berührungsflächen und damit auch die Anziehungskräfte zwischen den geprägten Aluminiumpigmenten könnten in diesem Fall sehr hoch sein.

Bei einer "Berg-zu-Berg"-Anordnung ist der Abstand der Aluminiumpigmente zueinander maximal, wobei die Berührungsflächen bzw. Berührungspunkte zugleich minimal sind. Bei dieser Anordnung der geprägten Aluminiumpigmente ist die Agglomerationsgefahr stark verringert.

Es hat sich bei der vorliegenden Erfindung gezeigt, dass die Agglomerationsneigung in der kosmetischen Zusammensetzung, vorzugsweise einem Nagellack oder einem Lipgloss, überraschend gering ist oder sogar gar nicht auftritt.

Der Fachmann würde erwarten, dass es in einem Medium mit erhöhter Viskosität zu einer Agglomeration der Aluminiumpigmente kommen muss, wie es auch bei glatten PVD-Aluminiumpigmenten ohne Prägung, beispielsweise Metalure® (erhältlich bei Fa. Eckart, Fürth, Deutschland), der Fall ist.

Es wird vermutet, daß sich die geprägten Aluminiumpigmente in der kosmetischen Zusammensetzung überwiegend in der "Berg-zu-Berg"-Anordnung anordnen. Bei der "Berg-zu-Berg"-Anordnung ist zwischen den geprägten Aluminiumpigmenten ausreichend Flüssigphase, beispielsweise Wasser, organisches Lösemittel, Öl, Wachs, etc. und/oder Dispergiermittel angeordnet, um der unerwünschten Agglomeration weiter entgegenzuwirken. Darüber hinaus wird vermutet, dass die geprägten Aluminiumpigmente in einer Anordnung vorliegen, bei der die Berglinien nicht parallel zueinander angeordnet sind, sondern in einer einander kreuzenden Anordnung vorliegen, was einer Stapelung und mithin einer Agglomeration der Aluminiumpigmente entgegenwirkt.

Vermutlich aufgrund dieser "Berg-zu-Berg"-Anordnung ist auch die Bereitstellung einer kosmetischen Zusammensetzung, die regelmäßig eine erhöhte Viskosität aufweist und zugleich geprägte Aluminiumpigmente enthält, möglich. Die geprägte Struktur, d.h. die Beugungsstrukturen, scheinen der Agglomeration der Pigmente entgegenzuwirken. Insofern ist es möglich, stabile kosmetische Zusammensetzungen, insbesondere Nagellack-Zusammensetzungen und die nachstehend beschriebenen Lipgloss-Zusammensetzungen zu formulieren.

Lipgloss soll den Lippen ein feuchtes, verführerisches Aussehen verschaffen. Lipgloss Zusammensetzungen enthalten im weitesten Sinn Öle und Wachse verschiedener Typen. Der ölige Bestandteil gibt der Zusammensetzung eine gewisse flüssige Konsistenz, während die Wachse den Schmelzpunkt erhöhen und für physikalische Stabilität sorgen. Lipgloss soll einen hohen Glanz aufweisen und auf den Lippen nicht verlaufen. Des weiteren sollten die Farbpigmente am Rand der Lippen nicht ausbluten. Ein gutes Hautgefühl ist ebenfalls erwünscht. Speziell bei Verwendung plättchenförmiger Pigmente impliziert dies, dass die Pigmente innerhalb der Zusammensetzung nicht agglomerieren dürfen.

Typische in Lipgloss-Zusammensetzungen verwendete Wachse sind Carnauba-Wachs, Candelilla-Wachs, Ozokerit, Ceresin, mikrokristallines Wachs, synthetische Wachse wie die Glycerinester der C18- bis C36-Fettsäuren, ferner Cetylalkohol, Stearylalkohol, Lanolin und Bienenwachs. Hierbei sorgt beispielsweise Ozokerit und insbesondere Bienenwachs für Festigkeit und Lanolin sorgt für Homogenität der Zusammensetzung während des Herstellungsprozesses und ebenfalls für ein gutes Hautgefühl.

Als Öle werden beispielsweise Rizinusöl, Canolaöl, Lanolinöl, Siliconöle, Polybuten, Mineralöle, Oleylalkohol, Isocetylalkohol und Ester wie Isopropylmyristat oder Decyloleat sowie Mischungen hieraus verwendet. Bevorzugt ist Rizinusöl, welches Farbstoffe löst und ebenfalls für ein gutes Hautgefühl sorgt.

Lipgloss-Zusammensetzungen mit metallischem Effekt sind eher ungewöhnlich. Lippen haben üblicherweise eine raue Struktur mit feinen Rissen. Derartige Strukturen werden beim Auftragen von Metallpigmenten wie beispielsweise ebenen Aluminiumpigmenten eher verstärkt und führen letztlich zu einem grauen und unattraktiven Erscheinungsbild.

Bei den erfindungsgemäßen Lipgloss Zusammensetzungen hingegen werden überraschenderweise interessante irisierende Farbeffekte in Kombination mit dem metallischen Aussehen erhalten. Der Glanz der Zusammensetzungen ist hoch und eine Trennung in einzelne Bestandteile tritt nicht auf, so daß für einen Betrachter der auf den Lippen aufgebrachte Überzug vorzugsweise wie ein geschlossener Film erscheint.

Die Pigmentierungshöhe der geprägten Aluminiumpigmente in der Lipgloss-Zusammensetzung beträgt vorzugsweise 0,2 bis 2,0 Gew.-%, weiter bevorzugt 0,3 bis 1,0 Gew.-%, bezogen auf das Gesamtgewicht der Zusammensetzung.

Die geprägten Aluminiumpigmente liegen in einer verdünnten Suspension in einem organischen Lösemittel vor. Hierbei handelt es sich vorzugsweise um ein für Lippenstifte geeignetes Lösemittel. Bevorzugt liegen die geprägten Aluminiumpigmente in Butylglycol in einer Konzentration von 1 bis 15 Gew.-%, bevorzugt 3 bis 10 Gew.-% vor. Bei Zugabe zu den restlichen Komponenten bei Herstellung der kosmetischen Zusammensetzung wird dann die gewünschte Endkonzentration von etwa 0,05 bis etwa 5 Gew.-%, bezogen auf das Gesamtgewicht der kosmetischen Zusammensetzung, erreicht.

Gemäß einer bevorzugten Ausführungsform enthält die Lipgloss-Zusammensetzung zusätzlich Farbmittel wie beispielsweise Farbpigmente und/oder Farbstoffe.
Visuell reizvolle Effekte können durch Mischungen von Farbpigmenten mit den geprägten Aluminiumpigmenten eingestellt werden. Als Farbpigmente können hierbei alle für Lippenstifte zugelassenen Farbpigmente verwendet werden.

Die geprägten Aluminiumpigmente bewirken einen metallischen Glanz und irisierende Farben im Regenbogenspektrum. Dieser Farbeffekt ist besonders stark im direkten Sonnenlicht. Aufgrund der geringen Dicke der geprägten Aluminiumpigmente und Formfaktoren, d.h. dem Verhältnis von Länge zu sekundärer Schichtdicke, von üblicherweise 60 bis 120 ist der irisierende Farbeffekt deutlich sichtbar, ohne dass einzelne Teilchen in dem aufgetragenen Lipgloss deutlich sichtbar wären, mithin kein "Sparkle-Effekt" auftritt. Dies verleiht dem Lipgloss ein elegantes Aussehen mit dezentem mehrfarbigem Schillern.

Bezogen auf die primäre Schichtdicke liegen die Formfaktoren vorzugsweise in einem Bereich von 1 50 bis 1000, wobei die Größe der geprägten Aluminiumpigmente vorzugsweise 20 µm nicht wesentlich überschreitet.

Bei einer Mischung von Farbpigment und Effektpigment können sich diese einander gut ergänzen. Im Rahmen geeigneter Konzentrationen übertönt keines der einzelnen Pigmente den Effekt des jeweils anderen Pigmentes. Ein derartiger Lipgloss erscheint nicht mehr silbern, sondern in einer metallischen Farbe mit einem irisierenden Farbeffekt.

Die Farbpigmente werden vorzugsweise in Konzentrationen von 0,5 bis 5 Gew.-%, bevorzugt von 1 bis 2,5 Gew.-%, bezogen auf das Gesamtgewicht der kosmetischen Zusammensetzung, verwendet. Innerhalb dieser Grenzen ist deren färbende Wirkung stark genug, ohne die durch die geprägten Aluminiumpigmente hervorgerufenen Effekte zu übertönen.

Des weiteren können in der erfindungsgemäßen Zusammensetzung für einen Lipgloss Zusätze wie Parfüme, Antioxidantien, Lichtschutzmittel oder Konservierungsstoffe enthalten sein. Hier werden übliche im Stand der Technik bekannte Substanzen eingesetzt.

### Beispiele

### I. Nagellackzusammensetzung

### Erfindungsgemäßes Beispiel 1:

| **Stoff** | **Konzentration in Gew.-%** |
|---|---|
| Nitrocellulose | 15 |
| Ethylacetat | 21,15 |
| n-Butylacetat | 39% |
| Isopropylalkohol | 8 % |
| Polyesterbindemittel | 9 % |
| Dibutylphthalat | 5% |
| Campher | 1,7 % |
| Stearalkonium Bentonit | 0,9 % |
| Geprägtes Aluminiumpigment (bezogen auf Pigment) | 0,25 % |

### Herstellung:

Zuerst wurde das Stearalkonium Bentonit in Butylacetat dispergiert und mit einer Mischung aus 2:1 Isopropanol/H2O (30 Gew.-% bezogen auf Stearalkonium Bentonit) aktiviert. Anschließend wurden alle Ingredienzien außer dem geprägten Aluminiumpigment zugegeben und gut vermischt. Abschließend gab man das geprägte Aluminiumpigment in Form einer 5 Gew.-%-igen Dispersion in Ethylacetat unter minimaler Schereinwirkung durch Rühren hinzu, bis eine homogene Dispersion erhalten wurde. Das Ethylacetat in der fertigen Nagellackzusammensetzung stammte hauptsächlich aus dieser Dispersion.

### Vergleichsbeispiel 2:

| **Stoff** | **Konzentration in Gew.-%** |
|---|---|
| Nitrocellulose | 15 |
| Ethylacetat | 19,4 |
| n-Butylacetat | 36 % |
| Isopropylalkohol | 8 % |
| Polyesterbindemittel | 9 % |
| Dibutylphthalat | 5 % |
| Campher | 1,7 % |
| Stearalkoniumbentonit | 0,9 % |
| Spectraflair® | 5 % |

Herstellung: Analog zu Beispiel 1.

### Vergleichsbeispiel 3:

| **Stoff** | **Konzentration in Gew.-%** |
|---|---|
| Nitrocellulose | 15 |
| Ethylacetat | 22,4 |
| n-Butylacetat | 39 % |
| Isopropylalkohol | 8 % |
| Polyesterbindemittel | 9 % |
| Dibutylphthalat | 5 % |
| Campher | 1,7 % |
| Stearalkoniumbentonit | 0,9 % |
| METALURE® L-55350 (Fa. Eckart) (bezogen auf Pigment) | 0,1 % |

Herstellung: Analog zu Beispiel 1. METALURE® wird in Form einer 10-%igen Dispersion in Ethylacetat zugegeben.

### Vergleich und Beurteilung der Zusammensetzungen der Beispiele 1 bis 3:

Visuelles Erscheinungsbild der Beispiele 1-3 nach Auftrag auf einen künstlichen Fingernagel:
- Erfindungsgemäßes Beispiel 1:: Glänzend Silber mit leichtem Regenbogenfarbeneffekt, geschlossener Metallfilm ähnlich einem "flüssigen Metall".
- Vergleichsbeispiel 2:: Starker Silberglanz mit starkem Regenbogenfarbeneffekt, einzelne Teilchen sichtbar ("Sparkle"-Effekt).
- Vergleichsbeispiel 3:: Glänzendes Silber ohne jeglichen Regenbogenfarbeneffekt, geschlossener Metallfilm ähnlich einem "flüssigen Metall".

Die kosmetischen Zusammensetzungen gemäß dem erfindungsgemäßen Beispiel 1 und dem Vergleichsbeispiel 2 wiesen eine vergleichbare Deckkraft auf, was sich jeweils an einem starken Silberglanz nach Aufbringung auf künstliche Fingernägel zeigte. Zu der gleichen Beurteilung gelangte man nach Begutachtung von Rakelabzügen von Zusammensetzungen mit variierenden Aluminiumpigmentkonzentrationen.

Im Unterschied zu der Zusammensetzung gemäß Vergleichsbeispiel 2 wurde bei der Zusammensetzung gemäß dem erfindungsgemäßen Beispiel 1 nur 1/20 der Menge an Aluminiumpigment benötigt, was ein Beleg für die außerordentliche Deckkraft der geprägten Aluminiumpigmente ist. Da zur Herstellung der erfindungsgemäßen kosmetischen Zusammensetzung signifikant weniger Aluminiumpigment verwendet wird, sinken die Herstellungskosten.

### II. Lipgloss-Zusammensetzung

### Erfindungsgemäßes Beispiel 4:

| **Stoff** | **Konzentration in Gew.-%** |
|---|---|
| PEG-45/Dodecyl Glycol Copolymer | 5,5 % |
| Candelilla-Wachs | 1,0 % |
| Hydroxyoctacosanyl-hydroxystearat | 9,9 % |
| Mikrokristallines Wachs | 5,6 % |
| Isopropyllanolat | 5 % |
| Rizinusöl | 48 % |
| Butylenglycol | 22,5 % |
| Geprägtes Aluminiumpigment | 2,5 % |

### Herstellung:

Die ersten sechs Bestandteile der vorstehend angegebenen Zusammensetzung für das erfindungsgemäße Beispiel 4 wurden mit einem Mischer mit niedriger Scherrate vermischt. Anschließend erhitzte man bis auf eine Temperatur von 80 - 84 °C und gab geprägtes Aluminiumpigment in Form einer 10%-igen Suspension in Propylenglycol hinzu, bis eine homogene Zusammensetzung erhalten wurde. Anschließend ließ man die aufgeschmolzene Masse durch eine 3-Rollenmühle passieren, erhitzte erneut und goß die Masse in eine vorgegebene Form und ließ die Masse auf Umgebungstemperatur abkühlen.

Die Herstellung der Zusammensetzung des nachstehenden erfindungsgemäßen Beispiels 5 bzw. der Zusammensetzungen der nachstehenden Vergleichsbeispiele 6 bis 9 erfolgte wie in analoger Weise wie beim erfindungsgemäßen Beispiel 4 beschrieben.

### Erfindungsgemäßes Beispiel 5:

| **Stoff** | **Konzentration in Gew.-%** |
|---|---|
| PEG-45/Dodecylglycol-Copolymer | 7,0 % |
| Candelilla-Wachs | 1,5 % |
| Hydroxyoctacosanyl-hydroxystearat | 10,9 % |
| Mikrokristallines Wachs | 6,3 % |
| Isopropyllanolat | 7 % |
| Rizinusöl | 54,5 % |
| Butylenglycol | 9 % |
| Rotes Farbpigment Rot Cosmetic D&C Red Nr. 7 C19-011 (Fa. Sun Chemical Corp., USA) | 2,9 % |
| Geprägtes Aluminiumpigment | 0,9% |

### Vergleichsbeispiel 6:

| **Stoff** | **Konzentration in Gew.-%** |
|---|---|
| PEG-45/Dodecylglycol-Copolymer | 5,5 % |
| Candelilla-Wachs | 1,0 % |
| Hydroxyoctacosanyl-hydroxystearat | 9,9 % |
| Mikrokristallines Wachs | 5,6 % |
| Isopropyllanolat | 5 % |
| Rizinusöl | 63,1 % |
| Butylenglycol | 9,0 % |
| Metalure L 55350 (bezogen auf Pigment) | 0,9 % |

### Vergleichsbeispiel 7:

| **Stoff** | **Konzentration in Gew.-%** |
|---|---|
| PEG-45/Dodecyl Glycol Copolymer | 5,5 % |
| Candelilla-Wachs | 1,0 % |
| Hydroxyoctacosanyl-hydroxystearat | 9,9 % |
| Mikrokristallines Wachs | 5,6 % |
| Isopropyllanolat | 5 % |
| Rizinusöl | 60,2 % |
| Butylenglycol | 9 % |
| Rotes Farbpigment Rot Cosmetic D&C Red Nr. 7 C19-011 (Fa. Sun Chemical, USA) | 2,9% |
| Metalure L 55350 (bezogen auf Pigment) | 0,9 % |

### Vergleichsbeispiel 8:

| **Stoff** | **Konzentration in Gew.-%** |
|---|---|
| PEG-45/Dodecyl Glycol Copolymer | 5,5 % |
| Candelilla-Wachs | 1,0 % |
| Hydroxyoctacosanyl-hydroxystearat | 9,9 % |
| Mikrokristallines Wachs | 5,6 % |
| Isopropyllanolat | 5 % |
| Rizinusöl | 68 % |
| Spectraflair® | 5,0 % |

### Vergleichsbeispiel 9:

| **Stoff** | **Konzentration in Gew.-%** |
|---|---|
| PEG-45/Dodecyl Glycol Copolymer | 7,0 % |
| Candelilla-Wachs | 1,5% |
| Hydroxyoctacosanyl-hydroxystearat | 10,9% |
| Mikrokristallines Wachs | 6,3 % |
| Isopropyllanolat | 7 % |
| Rizinusöl | 59,4 % |
| | |
| Rotes Farbpigment Rot Cosmetic D&C Red Nr. 7 C19-011 (Fa. Sun Chemical) | 2,9 % |
| Spectraflair® | 5 % |

### Vergleich und Beurteilung der Zusammensetzungen der Beispiele 4 bis 9:

Das Ergebnis des Vergleichs und die Beurteilung sind in Tabelle 1 zusammengefasst.

**Tabelle 1: Ergebnisse Lipgloss**

| **Probe** | **Erscheinungsbild Lipgloss Zusammensetzung** | **Erscheinungsbild nach Lipgloss-Auftrag** |
|---|---|---|
| Erfindungsgemäßes Beispiel 4 | Homogen, keine Agglomerate | Silber metallisch, irisierende Farben besonders im Sonnenlicht; keine individuellen Teilchen sichtbar, visuell geschlossener Film |
| Erfindungsgemäßes Beispiel 5 | Homogen, keine Agglomerate | Rötlich metallisch, irisierende Farben besonders im Sonnenlicht; keine individuellen Teilchen sichtbar, visuell geschlossener Film |
| Vergleichsbeispiel 6 | Homogen, Agglomerate | Grau; keine irisierende Farben; auch nicht im Sonnenlicht |
| Vergleichsbeispiel 7 | Homogen, Agglomerate | Grau mit schwacher rötlicher Farbe; keine irisierende Farben; auch nicht im Sonnenlicht |
| Vergleichsbeispiel 8 | Homogen, keine Agglomerate | Silber metallisch, irisierende Farben besonders im Sonnenlicht; individuelle Teilchen sichtbar ("Sparkle-Effekt"), kein geschlossener Film |
| Vergleichsbeispiel 9 | Homogen, keine Agglomerate | Rötlich metallisch, irisierende Farben besonders im Sonnenlicht; individuelle Teilchen sichtbar ("Sparkle-Effekt"), kein geschlossener Film |

## Patentansprüche

1. Kosmetische Zusammensetzung zur Erzeugung eines kosmetischen Überzugs mit einem metallisch und mehrfarbig schillernden Erscheinungsbild, umfassend eine Flüssigphase und PVD-Aluminiumpigment, wobei das PVD-Aluminiumpigment Beugungsstrukturen mit etwa 5.000 bis etwa 20.000 Strukturelementen pro cm, einen metallischen Aluminiumanteil von 90 bis 100 Gew.-%, bezogen auf das Gewicht des Aluminiumpigments, besitzt und in der kosmetischen Zusammensetzung in einer Pigmentierungshöhe von 0,05 bis 5,0 Gew.-%, bezogen auf das Gesamtgewicht der kosmetischen Zusammensetzung, enthalten ist.

2. Kosmetische Zusammensetzung nach Anspruch 1,
**dadurch gekennzeichnet,**
**daß** das Aluminiumpigment etwa 7.000 bis etwa 15.000 Strukturelemente pro cm aufweist.

3. Kosmetische Zusammensetzung nach Anspruch 1 oder 2,
**dadurch gekennzeichnet,**
**daß** die kosmetische Zusammensetzung zusätzlich wenigstens eine filmbildende Komponente enthält und ein Nagellack ist.

4. Kosmetische Zusammensetzung nach Anspruch 1 oder 2,
**dadurch gekennzeichnet,**
**daß** die kosmetische Zusammensetzung zusätzlich ein oder mehrere Wachse enthält und eine Lipgloss-Zusammensetzung ist.

5. Kosmetische Zusammensetzung nach Anspruch 3,
**dadurch gekennzeichnet,**
**daß** die filmbildende Komponente Nitrocellulose umfasst.

6. Kosmetische Zusammensetzung nach Anspruch 3 oder 5,
**dadurch gekennzeichnet,**
**daß** die filmbildende Komponente Nitrocellulose mit einem Molekulargewicht von mehr als 56.000 g/mol, vorzugsweise von mehr als 112.000 g/mol, umfasst.

7. Kosmetische Zusammensetzung nach einem der Ansprüche 3, 5 oder 6,
**dadurch gekennzeichnet,**
**daß** die filmbildende Komponente Celluloseacetatbutyrat, das vorzugsweise ein Molekulargewicht von mehr als 83000 g/mol, umfasst.

8. Kosmetische Zusammensetzung nach einem der Ansprüche 3, 5, 6 oder 7,
**dadurch gekennzeichnet,**
**daß** die Flüssigphase ein organisches Lösemittel ist, das vorzugsweise aus der Gruppe, die aus Toluol, n-Propylacetat, iso-Propylacetat, iso-Propylalkohol, Ethylacetat, Ethanol, Glycolether, n-Butylacetat, Methylproposolacetat und Gemischen daraus besteht, ausgewählt wird.

9. Kosmetische Zusammensetzung nach einem der Ansprüche 3, 5, 6, 7 oder 8,
**dadurch gekennzeichnet**,
das die kosmetische Zusammensetzung zusätzlich einen Weichmacher und/oder ein Dispergierungsmittel enthält.

10. Kosmetische Zusammensetzung nach einem der Ansprüche 3, 5, 6, 7, 8 oder 9,
**dadurch gekennzeichnet**,
das die kosmetische Zusammensetzung als Weichmacher wenigstens Dibutylphthalat, gegebenenfalls in Kombination mit Saccharoseacetatisobutyrat, enthält.

11. Kosmetische Zusammensetzung nach einem der Ansprüche 9 oder 10,
**dadurch gekennzeichnet**,
das die kosmetische Zusammensetzung als Dispergiermittel Bentonite und/oder polymeren Harnstoff, enthält.

12. Kosmetische Zusammensetzung nach einem der Ansprüche 3, 5, 6, 7, 8, 9, 10 oder 11,
**dadurch gekennzeichnet**,
Aluminiumpigment in einer Pigmentierungshöhe von 0,1 bis 2,0 Gew.-% enthalten ist.

13. Kosmetische Zusammensetzung nach einem der Ansprüche 3, 5, 6, 7, 8, 9, 10, 11 oder 12,
**dadurch gekennzeichnet,**
**daß** die kosmetische Zusammensetzung zusätzlich mindestens ein Farbmittel enthält.

14. Kosmetische Zusammensetzung nach Anspruch 13,
**dadurch gekennzeichnet,**
**daß** das Farbmittel ein Farbpigment und/oder Farbstoff ist.

15. Kosmetische Zusammensetzung nach einem der Ansprüche 3, 5, 6, 7, 8, 9, 10, 11, 12, 13 oder 14,
**dadurch gekennzeichnet,**
**daß** die Beugungsstrukturen ein Reflexionsgitter sind.

16. Kosmetische Zusammensetzung nach einem der Ansprüche 3, 5, 6, 7, 8, 9, 10, 11, 12, 13, 14 oder 15,
**dadurch gekennzeichnet,**
**daß** das PVD-Aluminiumpigment eine Schichtdicke von etwa 20 nm bis etwa 140 nm, vorzugsweise von etwa 20 nm bis etwa 100 nm, aufweist.

17. Kosmetische Zusammensetzung nach Anspruch 4,
**dadurch gekennzeichnet,**
**daß** die Flüssigphase zusätzlich Öl und/oder Gel enthält.

18. Kosmetische Zusammensetzung nach Anspruch 4 oder 17,
**dadurch gekennzeichnet,**
**daß** die kosmetische Zusammensetzung Zusatzstoffe wie Antioxidantien, Farbmittel, Emulgatoren, Vitamine, Duftstoffe und/oder Füllstoffe enthält.

19. Kosmetische Zusammensetzung nach Anspruch 17 oder 18,
**dadurch gekennzeichnet,**
**daß** das Öl aus der Gruppe, die aus Rizinusöl, Canolaöl, Lanolinöl, Siliconöle, Polybuten, Mineralöle, Oleylalkohol, Isocetylalkohol und Ester wie Isopropylmyristat oder Decyloleat und Mischungen davon besteht, ausgewählt wird.

20. Kosmetische Zusammensetzung nach einem der Ansprüche 4, 17, 18 oder 19,
**dadurch gekennzeichnet,**
**daß** das Wachs aus der Gruppe, die aus Carnauba-Wachs, Candelilla-Wachs, Ozokerit, Ceresin, mikrokristallinem Wachs, synthetischen Wachse wie die Glycerinester der C18- bis C36-Fettsäuren, Cetylalkohol, Stearylalkohol, Lanolin, Bienenwachs und Mischungen davon besteht, ausgewählt wird.

21. Kosmetische Zusammensetzung nach einem der Ansprüche 4, 17, 18, 19, oder 20,
**dadurch gekennzeichnet,**
**daß** die kosmetische Zusammensetzung zusätzlich mindestens ein Farbmittel enthält.

22. Kosmetische Zusammensetzung nach Anspruch 21,
**dadurch gekennzeichnet,**
**daß** das Farbmittel ein Farbpigment und/oder Farbstoff ist.

23. Kosmetische Zusammensetzung nach einem der Ansprüche 4, 17, 18, 19, 20, 21 oder 22,
**dadurch gekennzeichnet,**
**daß** die kosmetische Zusammensetzung weiterhin einen oder mehrere Zusätze wie Parfümes, Antioxidantien, Lichtschutzmittel oder Konservierungsstoffe enthalten.

24. Kosmetische Zusammensetzung nach einem der Ansprüche 4, 17, 18, 19, 20, 21, 22 oder 23,
**dadurch gekennzeichnet,**
**daß** das Aluminiumpigment in einer Pigmentierungshöhe von 0,2 bis 2,0 Gew.-%, bezogen auf das Gesamtgewicht der kosmetischen Zusammensetzung, enthalten ist.

25. Kosmetische Zusammensetzung nach einem der Ansprüche 4, 17, 18, 19, 20, 21, 22 ,23 oder 24,
**dadurch gekennzeichnet,**
**daß** die Beugungsstrukturen ein Reflexionsgitter sind.

26. Kosmetische Zusammensetzung nach einem der Ansprüche 4, 17, 18, 19, 20, 21, 22, 23, 24 oder 25,
**dadurch gekennzeichnet,**
**daß** das PVD-Aluminiumpigment eine Schichtdicke von etwa 20 nm bis etwa 140 nm, vorzugsweise von etwa 20 nm bis etwa 100 nm, aufweist.

27. Künstliche Fingernägel,
**dadurch gekennzeichnet,**
**daß** die künstlichen Fingernägel mit einer kosmetischen Zusammensetzung nach einem der Ansprüche 3, 5, 6, 7, 8, 9, 10, 11, 12, 13, 14 oder 15 überzogen ist.

## Claims

1. Cosmetic composition for creating a cosmetic coating having a metallic and multicoloured iridescent appearance, comprising a liquid phase and a PVD aluminium pigment, wherein said PVD aluminium pigment has diffractive structures containing from approximately 5,000 to approximately 20,000 structural elements per cm and a metallic aluminium content of from 90 to 100% by weight, based on the weight of the aluminium pigment, and is present in the cosmetic composition at a pigmentation level of from 0.05 to 5.0% by weight, based on the total weight of the cosmetic composition.

2. Cosmetic composition according to claim 1, **characterized in that** the aluminium pigment has from approximately 7,000 to approximately 15,000 structural elements per cm.

3. Cosmetic composition according to claim 1 or 2, **characterized in that** the cosmetic composition additionally contains at least one film-forming component and is a nail varnish.

4. Cosmetic composition according to claim 1 or 2, **characterized in that** the cosmetic composition additionally contains one or more waxes and is a lip gloss composition.

5. Cosmetic composition according to claim 3, **characterized in that** the film-forming component comprises cellulose nitrate.

6. Cosmetic composition according to claim 3 or 5, **characterized in that** the film-forming component comprises cellulose nitrate having a molecular weight of more than 56,000 g/mol, preferably of more than 112,000 g/mol.

7. Cosmetic composition according to any of claims 3, 5 or 6, **characterized in that** the film-forming component comprises cellulose acetate butyrate, preferably having a molecular weight of more than 83,000 g/mol.

8. Cosmetic composition according to any of claims 3, 5, 6 or 7, **characterized in that** the liquid phase is an organic solvent, which is preferably selected from the group consisting of toluene, n-propyl acetate, isopropyl acetate, isopropyl alcohol, ethyl acetate, ethanol, glycol ether, n-butyl acetate, methyl proposol acetate, and mixtures thereof.

9. Cosmetic composition according to any of claims 3, 5, 6, 7 or 8, **characterized in that** the cosmetic composition additionally contains a plasticizer and/or a dispersing agent.

10. Cosmetic composition according to any of claims 3, 5, 6, 7, 8 or 9, **characterized in that** the cosmetic composition contains, as plasticizer, at least dibutyl phthalate, optionally together with saccharose acetate isobutyrate.

11. Cosmetic composition according to either of claims 9 and 10, **characterized in that** the cosmetic composition contains, as dispersing agent, bentonites and/or polymeric urea.

12. Cosmetic composition according to any of claims 3, 5, 6, 7, 8, 9, 10 or 11, **characterized in that** the aluminium pigment is present at a pigmentation level of from 0.1% to 2.0% by weight.

13. Cosmetic composition according to any of claims 3, 5, 6, 7, 8, 9, 10, 11 or 12, **characterized in that** the cosmetic composition additionally contains at least one colouring agent.

14. Cosmetic composition according to claim 13, **characterized in that** the colouring agent is a coloured pigment and/or a dye.

15. Cosmetic composition according to any of claims 3, 5, 6, 7, 8, 9, 10, 11, 12, 13 or 14, **characterized in that** the diffractive structures are a reflection grating.

16. Composition according to any of claims 3, 5, 6, 7, 8, 9, 10, 11, 12, 13, 14 or 15, **characterized in that** the PVD aluminium pigment has a layer thickness of from approximately 20 nm to approximately 140 nm, preferably from approximately 20 nm to approximately 100 nm.

17. Cosmetic composition according to claim 4, **characterized in that** the liquid phase additionally contains oil and/or gel.

18. Cosmetic composition according to claim 4 or 17, **characterized in that** the cosmetic composition contains additives such as antioxidants, colouring agents, emulsifiers, vitamins, perfumes and/or fillers.

19. Cosmetic composition according to claim 17 or 18, **characterized in that** the oil is selected from the group consisting of castor oil, canola oil, lanoline oil, silicone oils, polybutene, mineral oils, oleyl alcohol, isocetyl alcohol and esters such as isopropyl myristate or decyl oleate, and mixtures thereof.

20. Cosmetic composition according to any of claims 4, 17, 18 or 19, **characterized in that** the wax is selected from the group consisting of carnauba wax, candelilla wax, ozocerite, ceresine, microcrystalline wax, synthetic waxes such as the glycerin esters of C18 to C36 fatty acids, cetyl alcohol, stearyl alcohol, lanoline, beeswax, and mixtures thereof.

21. Cosmetic composition according to any of claims 4, 17, 18, 19 or 20, **characterized in that** the cosmetic composition additionally contains at least one colouring agent.

22. Cosmetic composition according to claim 21, **characterized in that** the colouring agent is a coloured pigment and/or a dye.

23. Cosmetic composition according to any of claims 4, 17, 18, 19, 20, 21 or 22, **characterized in that** the cosmetic composition further contains one or more additives such as perfumes, antioxidants, light-stabilizing agents or preservatives.

24. Cosmetic composition according to any of claims 4, 17, 18, 19, 20, 21, 22 or 23, **characterized in that** the aluminium pigment is present at a pigmentation level of from 0.2 to 2.0% by weight, based on the total weight of the cosmetic composition.

25. Cosmetic composition according to any of claims 4, 17, 18, 19, 20, 21, 22, 23 or 24, **characterized in that** the diffractive structures are a reflection grating.

26. Cosmetic composition according to any of claims 4, 17, 18, 19, 20, 21, 22, 23, 24, or 25, **characterized in that** the PVD aluminium pigment has a layer thickness of from approximately 20 nm to approximately 140 nm, preferably from approximately 20 nm to approximately 100 nm.

27. Artificial fingernail, **characterized in that** the artificial fingernail is coated with a cosmetic composition according to any of claims 3, 5, 6, 7, 8, 9, 10, 11, 12, 13, 14 or 15.

## Revendications

1. Composition cosmétique pour produire une pellicule cosmétique d'aspect miroitant métallique et polychrome, comprenant une phase liquide et un pigment d'aluminium-PVD, le pigment d'aluminium-PVD possédant des structures de diffraction comportant environ 5000 à environ 20 000 éléments structuraux par cm, une teneur en aluminium métallique de 90 à 100 % en poids par rapport au poids du pigment d'aluminium et étant contenu dans la composition cosmétique à une hauteur de pigmentation de 0,05 à 5,0 % en poids par rapport au poids total de la composition cosmétique.

2. Composition cosmétique selon la revendication 1,
**caractérisé en ce**
**que** le pigment d'aluminium présente environ 7000 à environ 15 000 éléments structuraux par cm.

3. Composition cosmétique selon la revendication 1 ou 2,
**caractérisée en ce**
**que** la composition cosmétique contient en outre au moins un composant filmogène et est un vernis à ongle.

4. Composition cosmétique selon la revendication 1 ou 2,
**caractérisée en ce**
**que** la composition cosmétique contient en outre une ou plusieurs cires et est une composition de brillant à lèvres.

5. Composition cosmétique selon la revendication 3,
**caractérisée en ce**
**que** le composant filmogène comprend de la nitrocellulose.

6. Composition cosmétique selon la revendication 3 ou 5,
**caractérisée en ce**
**que** le composant filmogène comprend de la nitrocellulose présentant un poids moléculaire supérieur à 56 000 g/mole, de préférence supérieur à 112 000 g/mole.

7. Composition cosmétique selon l'une quelconque des revendications 3, 5 ou 6,
**caractérisée en ce**
**que** le composant filmogène comprend de l'acétate-butyrate de cellulose qui présente de préférence un poids moléculaire supérieur à 83 000 g/moles.

8. Composition cosmétique selon l'une quelconque des revendications 3, 5, 6 ou 7,
**caractérisée en ce**
**que** la phase liquide est un solvant organique qui est choisi de préférence dans le groupe comprenant le toluène, le n-propylacétate, l'iso-propylacétate, l'alcool d'iso-propyle, l'éthylacétate, l'éthanol, l'éther de glycol, le n-butylacétate, le méthylproposolacétate et leurs mélanges.

9. Composition cosmétique selon l'une quelconque des revendications 3, 5, 6, 7 ou 8,
**caractérisée en ce**
**que** la composition cosmétique contient en outre un plastifiant et/ou un agent dispersant.

10. Composition cosmétique selon l'une quelconque des revendications 3, 5, 6, 7, 8 ou 9,
**caractérisée en ce**
**que** la composition cosmétique contient comme plastifiant, au moins du dibutylphtalate, éventuellement en combinaison avec de l'acétate-isobutyrate de saccharose.

11. Composition cosmétique selon l'une quelconque des revendications 9 ou 10,
**caractérisée en ce**
**que** la composition cosmétique contient comme agent dispersant, de la bentonite et/ou de l'urée polymère.

12. Composition cosmétique selon l'une quelconque des revendications 3, 5, 6, 7, 8, 9, 10 ou 11,
**caractérisée en ce**
**que** le pigment d'aluminium est contenu à une hauteur de pigmentation de 0,1 à 2,0 % en poids.

13. Composition cosmétique selon l'une quelconque des revendications 3, 5, 6, 7, 8, 9, 10, 11 ou 12,
**caractérisée en ce**
**que** la composition cosmétique contient en outre au moins un agent colorant.

14. Composition cosmétique selon la revendication 13,
**caractérisée en ce**
**que** l'agent colorant est un pigment et/ou un colorant.

15. Composition cosmétique selon l'une quelconque des revendications 3, 5, 6, 7, 8, 9, 10, 11, 12, 13 ou 14,
**caractérisée en ce**
**que** les structures de diffraction sont un réseau de réflexion.

16. Composition cosmétique selon l'une quelconque des revendications 3, 5, 6, 7, 8, 9, 10, 11, 12, 13, 14 ou 15,
**caractérisée en ce**
**que** le pigment d'aluminium-PVD présenté une épaisseur de couche d'environ 20 nm à environ 140 nm, de préférence d'environ 20 nm à environ 100 nm.

17. Composition cosmétique selon la revendication 4,
**caractérisée en ce**
**que** la phase liquide contient en outre de l'huile et/ou un gel.

18. Composition cosmétique selon la revendication 4 ou 17,
**caractérisée en ce**
**que** la composition cosmétique contient des additifs tels que des anti-oxydants, colorants, émulsifiants, vitamines, parfums et/ou substances de remplissage.

19. Composition cosmétique selon la revendication 17 ou 18,
**caractérisée en ce**
**que** l'huile est choisie dans le groupe comprenant l'huile de ricin, l'huile de canola, l'huile de lanoline, l'huile de silicone, le polybutène, les huiles minérales, l'alcool d'oléyle, l'alcool d'isocétyle et les esters tels que l'isopropylmyristate ou le décyloléate et leurs mélanges.

20. Composition cosmétique selon l'une quelconque des revendications 4, 17, 18 ou 19,
**caractérisée en ce**
**que** la cire est choisie dans le groupe comprenant la cire de carnauba, la cire de candelilla, l'ozocérite, la cérésine, la cire microcristalline, les cires synthétiques telles que les esters de glycérine d'acides gras en C₁₈ à C₃₆, l'alcool de cétyle, l'alcool de stéaryle, la lanoline, la cire d'abeille et leurs mélanges.

21. Composition cosmétique selon l'une quelconque des revendications 4, 17, 18, 19 ou 20,
**caractérisée en ce**
**que** la composition cosmétique contient en outre au moins un agent colorant.

22. Composition cosmétique selon la revendication 21,
**caractérisée en ce**
**que** l'agent colorant est un pigment et/ou un colorant.

23. Composition cosmétique selon l'une quelconque des revendications 4, 17, 18, 19, 20, 21 ou 22,
**caractérisée en ce**
**que** la composition cosmétique contient en outre un ou plusieurs additifs tels que parfums, anti-oxydants, agents de protection contre la lumière ou conservateurs.

24. Composition cosmétique selon l'une quelconque des revendications 4, 17, 18, 19, 20, 21, 22 ou 23,
**caractérisée en ce**
**que** le pigment d'aluminium est contenu à une hauteur de pigmentation de 0,2 à 2,0 % en poids par rapport au poids total de la composition cosmétique.

25. Composition cosmétique selon l'une quelconque des revendications 4, 17, 18, 19, 20, 21, 22, 23 ou 24,
**caractérisée en ce**
**que** les structures de diffraction sont un réseau de réflexion.

26. Composition cosmétique selon l'une quelconque des revendications 4, 17, 18, 19, 20, 21, 22, 23, 24 ou 25,
**caractérisée en ce**
**que** le pigment d'aluminium-PVD présente une épaisseur de couche d'environ 20 nm à environ 140 nm, de préférence d'environ 20 nm à environ 100 nm.

27. Ongle artificiel,
**caractérisé en ce**
**que** l'ongle artificiel est revêtu d'une composition cosmétique selon l'une quelconque des revendications 3, 5, 6, 7, 8, 9, 10, 11, 12, 13, 14 ou 15.
